# EUROPEAN PATENT SPECIFICATION

(11) **EP 1 408 988 B1**
(45) Date of publication and mention of the grant of the patent: **13.06.2007**
(21) Application number: 00976861.5
(22) Date of filing: 02.11.2000
(51) Int. Cl.: A61K 45/06, A61K 31/715, A61K 31/10, A61K 31/7008, A61P 19/04, A61P 19/02

(54) **COMPOSITIONS OF ORALLY ADMINISTERED NUTRITIONAL SUPPLEMENTS TO REPAIR ARTICULAR CARTILAGE**
ORAL ZU VERABREICHENDE ZUBEREITUNGEN VON NAHRUNGSMITTELZUSÄTZEN ZUR HEILUNG VON GELENKKNORPELN
COMPOSITION DE SUPPLEMENTS ADMINISTREES PAR VOIE ORALE POUR REPARER LE CARTILAGE ARTICULAIRE

(30) Priority: 02.11.1999 US 162948 P
(43) Date of publication of application: 21.04.2004
(73) Proprietor: Madere, Shawn Paul, Paris, KY 40361 (US)
(72) Inventor: Madere, Shawn Paul, Paris, KY 40361 (US)
(74) Representative: Dunlop, Brian Kenneth Charles
(86) International application number: PCT/US2000/030268
(87) International publication number: WO 2001/032188

(56) References cited:
- EP-A- 0 214 642
- WO-A-98/52556
- US-A- 5 364 845
- US-A- 5 843 923
- US-A- 5 902 801
- US-A- 5 916 565
- US-A- 6 136 795

## Description

This application claims the benefit of priority in U.S. Provisional Application Serial Number: 60/162,948, filed November 2,1999.

### Field of Invention

The present invention is directed to compositions of nutritional supplements. Specifically, the invention relates to a unique compound that, when ingested by mammals, is capable of repairing and regenerating articular cartilage, resulting in relief from associated symptoms of articular cartilage degeneration.

### Background of the Invention

The connective joints of mammals can be categorized as one of the following types:
(1) Fixed or structured joints such as those found in the plates of the skull;
(2) Amphiarthroidal or limited mobility joints such as those of the pelvis or sacroiliac; and
(3) Diarthroidal or Synovial joints. These joints are highly mobile consisting of, hinge, ball and socket, saddle or gliding joints; such as knee, hip, and carpal joints. These highly mobile joints have similar structures and components including, "the joint capsule," this is the outer membrane, which encases the joint and connects one bone to the other. "The Synovium" is the inner lining of the joint capsule which secretes synovial fluid. Molecules of hyalauronic acid are responsible for the viscosity of the synovial fluid and play a crucial role in maintaining healthy cartilage and protecting the joint surface. Articular cartilage is a matrix of proteoglycans, chondrocytes, and collagen, which caps the articulating ends of the bones. Articular cartilage absorbs shock from mechanical forces and provides a viscous surface so that bone ends may glide easily across one another. Ligaments, tendons, muscles, and bursae provide structure and stabilization to the joints as well. All must function properly together to insure ease of movement and longevity for the joint.

Our primary concern is the status of the articular cartilage. When healthy, articular cartilage forms a smooth, slick surface for the bone ends providing pain free movement and resilience. Many variables influence the viability of articular cartilage including, mechanical forces, trauma, disease, aging, and osteoarthritis. In all such cases, the integrity of the articular cartilage may be compromised. As the healthful qualities of articular cartilage diminish, wear is inevitable. Bone surfaces become coarse resulting in painful afflictions of inconvenience, inflammation, and in extreme cases incapacitation.

Articular cartilage is comprised of 65-80% water, collagen, proteoglycans, and chondrocytes. Collagen comprises microscopic fibers found in all tissues such as skin, tendons ligaments, and joint cartilage. This versatile protein provides elasticity and the structural framework of the cartilage matrix. Proteoglycans are molecules of protein and amino sugars, interwoven with collagen fibers to form the articular cartilage. Due to their dense negative ion content, these molecules are able to attract and retain water within the cartilage formation specifically for lubrication.

Proteoglycans provide the unique mechanical properties for resiliency and recovery under compressive forces. Chondrocytes are active cells within the cartilage, which manufacture new collagen and proteoglycan molecules while excreting enzymes, which remove damaged cartilage and proteoglycan molecules. Chondrocytes also produce synovial fluid for nutrient transportation and hyaluronan lubrication. Synovial cells work in concert with chondrocytes to produce hyaluronan; also known as hyaluronic acid. This important glycosaminoglycan (GAG) is an integral part of both synovial fluid and articular cartilage. Within the articular cartilage, hyaluronan provides viscoelastic properties allowing ease of motion between opposing surfaces and increasing compressive resistance. Within the synovium, hyaluronic acid as synovial fluid provides an effective barrier regulating the introduction of plasma components. Under normal conditions, the body will synthesize sufficient amounts of base components to maintain and regenerate articular cartilage, while limiting the production and release of destructive proteinases and enzymes. Yet, with the onset of degeneration, the demand for these base components becomes taxed and supplementation becomes necessary. Supplementation being the addition of metabolic precursors to the diet, aiding in the biosynthesis of proteoglycans, GAG's, hyaluron, and collagen.

Metabolic precursors for the production of articular cartilage include, chondroitin sulfate, a glycosaminoglycan polysaccharide, which is a primary component of articular cartilage comprising an aminosugar and an organic acid or sugar. Specifically chondroitin sulfate is broken down into sulfate disaccharides and N-Acetyl galactosamine. Glucuronic acid is the key substrate comprising one half of the hyaluronan molecule, the other being N-Acetyl D-glucosamine. Chondroitin sulfate, as CS4 and CS6 within the body, are a critical class of glycosaminoglycans which bind water to the articular cartilage matrix and are necessary for the formation of proteoglycans.

Glucosamine, as glucosamine 5-phosphate, is naturally occurring within the body. It is fundamental catalyst for the biosynthesis of glycosaminoglycans, proteoglycans, hyaluronan, and collagen. Glucosamine is the primary amino sugar found in tissues and articular cartilage. Glucosamine is available in exogenous forms, glucosamine sulfate sodium, glucosamine hydrochloride and N-Acetyl D-Glucosamine. These forms are highly bioavailable in mammals. The remaining exogenous form, glucosamine hydroiodide, is not well tolerated or assimilated.

Each compound demonstrates properties unique in origin and activity within the body. Glucosamine sulfate sodium, glucose and an amine bound to sodium sulfate, is made bioavailable by catalyzing the conversion of glucosamine to GAGs. Glucosamine hydrochloride, an amino derivative obtained by chemical hydrolysis of hydrochloric acid on disaccharides, is bioavailable for the production of hyaluronic acid, N-Acetyl D-Glucosamine, an amino derivative of glucose obtained by chemical hydrolysis of chitin, a polysaccharide and sub-component of hyaluronic acid. Glucosamine sulfate potassium, an aminosugar composition bound to the mineral potassium, facilitates cellular membrane function and sustained release of primary substrates (glucosamine) for collagen and proteoglycan synthesis. Methylsulfonylmethane (MSM), the most bio-available sulfur compound found in the body, is an integral part of hemoglobin and body tissue, and is essential for the synthesis of connecting tissues, collagen and the essential amino acids methionine and cysteine. Utilized as an anti-inflammatory and blood vessel dilator, exogenous MSM influences cellular membrane potentials relating to cellular transfer of sodium and potassium. Manganese proteinate is a peptide bound mineral which catalyzes GAG and collagen synthesis. Sodium ascorbate is an electrolyte bound ascorbate needed for collagen production and aids in the body's ability to utilize manganese.

The biosynthesis of these metabolic precursors follow specific pathways to produce new articular cartilage while regulating the damaging effects of destructive enzymes. Exogenous glucosamine allows the body to exceed the natural rate-limiting thresholds whereby glucosamine becomes the stimulant in the production of proteoglycans and GAGs. Exogenous glucosamine also stimulates the chondrocytes to produce more collagen and enhance articular cartilage metabolism. Once this cycle is enacted, the metabolic precursors are utilized until the rate-limiting threshold is normalized.

Numerous disclosures suggest the introduction of nutritional supplements as therapy for the treatment of connective tissues. For example, in U.S. Patent No. 3,682,076 (Rovati et al.) glucosamine sulfates are used to treat arthritic conditions. To U.S. Patent No. 3,697,652 (Rovati et al.), N-acetyl glucosamine is used to treat degenerative afflictions of the joints. U.S. Patent Nos. 5,364,845 and 5,587,363 (both to Hederson) show that glucosamine, chondroitin and manganese are used to protect and repair connective tissue. In U.S. Patent No. 5,840,715 (Florio), N-acetyl glucosamine sulfate, chondroitin sulfate, gamma linolenic acid eicosapentaenoic acid and docosahexaenoic acid, and manganese aspartate are combined to treat arthritis symptoms. U.S. Patent No. 5.916.565 (Rose et al) teaches a composition comprised of D-glucosamine hydrochloride, chondroitin sulfate, cayenne, ginger, turmeric, yucca, Devil's Claw, nettle leaf, Black Cohosh, alfalfa, and celery seeds to repair and maintain damaged tissues in joints of vertebrates. In U.S. Patent No. 5,162,303 (Goodman et al.), ascorbate is used as a collagen modifier and a wound and tissue healer. In U.S. Patent No. 5,922,692 (Marino), glucosamine sulfate and chondroitin sulfate are added to foodstuffs. Finally, in U.S. Patent No. 4,973,605 (Hershler), methylsulfonylmethane (MSM) is touted as an anti-inflammatory and pain reliever.

EP-A-0 214 642 discloses a pharmaceutically acceptable composition of glucosamine sulfate potassium.

Accordingly it is understood, the previous references have been useful to varying degrees; however, none of these prior investigators disclose a complete composition of metabolic precursors, comprising: glucosamine potassium, methylsulfonylmethane, chondroitin sulfate, glucosamine sulfate, glucosamine hydrochloride, N-Acetyl D-Glucosamine, sodium ascorbate, and manganese proteinate, specifically combined to work synergistically as biocatalyst in the production of articular cartilage. Nor do these prior disclosures teach or suggest the use of glucosamine sulphate potassium and methylsulfonylmethane to facilitate cellular uptake of these vital precursors.

### Summary of the Invention

The primary object of the present invention is the reparation of articular cartilage through oral introduction of a novel combination of metabolic precursors, wherein such precursors are nutritional supplements that are readily absorbed via ingestion. It is also a primary objective of the invention to offer a treatment by which these nutritional supplements may be ingested in order to produce a chondroprotective effect while assisting in the nutrient transfer within the cells of the articular cartilage.

The present invention is directed to a composition and accelerating the process of articular regeneration by:
A) providing exogenous chondroitin sulfate to stimulate the production of proteoglycans, glycosaminoglycans, and collagen, inhibit degenerative enzymes excreted by the chondrocytes, synovial cells, and aid in nutrient transportation within the synovial fluid;
B) providing exogenous chondroitin sulfate in concert with N-acetyl D-glucosamine; increasing the production and availablility of hyaluronan through the inclusion of its prime substrates galactosamine (through chondroitin sulfate assimilation) and N-Acetyl D-glucosamine;
C) providing exogenous glucosamine sulfate sodium to stimulate chondrocyte production of proteoglycans and glycosaminoglycans;
D) providing exogenous glucosamine hydrochloride and N-Acetyl D-Glucosamine to stimulate chondrocyte production of hyaluronic acid;
E) providing exogenous glucosamine sulfate potassium to facilitate cellular membrane function within the chondrocytes enhancing the availability of the existing glucosamines and stimulating the production of collagen and proteoglycans;
F) providing exogenous methylsulfonylmethane to regulate proper cellular membrane function by normalizing sulfur content within the body and facilitate the production of collagen, reduce pain and provide anti-inflammatory properties;
G) providing exogenous sodium absorbate to scavenge free radicals within the joint capsule, modify type II collagen, assist in the healing of related tissues and facilitate the utilization of manganese in the biosynthetic pathway; and
H) Provide exogenous manganese proteinate to catalyze the production of proteoglycans and collagen from glucosamine.

### Detailed Description of the Invention

The present invention discloses a novel composition of nutritional supplements adapted for oral ingestion, comprised of at least two specific metabolic precursors to the production of proteoglycans and collagen. The novel composition provided by the invention works synergistically to repair and replenish articular cartilage, resulting in symptomatic relief of joint pain and inflammation caused by articular cartilage degeneration. The critical inclusion of glucosamine sulfate potassium and methylsulfonylmethane provide a unique delivery system for the associated embodiments of this invention. Their ability to regulate cellular membrane function and nutrient transportation within the articular cartilage enhances the effectiveness of the related metabolic precursors.

The present invention provides glucosamine in all four bioavailable forms as a metabolic precursor to the production of proteoglycans, glycosaminoglycans, collagen, and hyaluronic acid. Previously patented compounds we are aware of have included, glucosamine sulfate, glucosamine hydrochloride, and N-Acetyl D-Glucosamine, alone or in a various combinations thereof. While these combinations are effective to varying degrees, a sodium imbalance is achieved, inhibiting full cellular volumizing of the available glucosamine.

The compositions provided by the present invention include glucosamine sulfate potassium in order to stabilize the ionic transfer of nutrients within the cells of the articular cartilage. Sodium ions outside the cell wall are dependent upon potassium ions inside the cell for maintaining the essential balance between tissue fluids. This ionic balance across the cell's outer membrane allows the proper movement of nutrients into and waste product removal out of the cell. Glucosamine sulfate potassium works in concert with glucosamine sulfate sodium activating the mechanism termed "sodium/potassium pump." Within this synergistic relationship, water, nutrients, and waste products transfer back and forth between the nucleoplasm, cytoplasm, and protoplasm of the cells. Without the proper balance of sodium and potassium cellular function is less than optimal.

Glucosamine sulfate potassium is hypothesized to be a key factor in achieving this balance, and in determining cellular uptake of glucosamine and chondroitin. Glucosamine sulfate potassium normalizes the sodium/potassium ratio thus achieving the proper physiological balance necessary for metabolic precursor utilization resulting in articular cartilage regeneration.

Methylsulfonylmethane (MSM) has unique properties which aid in the bioavailability of the present invention. MSM provides the essential amino acids, methionine and taurine, cystine and glutathione; necessary for proper cellular function. Methionine and Taurine enhance cell volumizing while regulating glucose metabolism. Cystine combines with glutamine to produce and maintain glutathione; the most abundant water-soluble antioxidant found inside tissue cells. This naturally occurring free radical fighting substrate protects the cells of the articular cartilage by inhibiting oxidation of the cartilage matrix. The addition of absorbate further enhances the positive effects of glutathione by elevating and maintaining intercellular levels of glutathione although the effects are indirect. The inclusion of methylsulfonylmethane in the embodiments of the present invention provides the necessary elements to protect the protoplasm of the cells and normalize membrane function.

In a preferred embodiment, the invention provides the unique combination of glucosamine sulfate potassium and methylsulfonylmethane in the compositions of the invention to enhance the effectiveness of the present invention by achieving equilibrium between the cellular fluids, thus increasing the bioavailablility of the metabolic precursors therein.

In another embodiment MSM and ascorbate are provided in combination in the compositions of the invention mentioned before on pages 7-9 to work synergistically as a chondro-protective antioxidant. Likewise, these compositions embodied by the invention further comprise manganese proteinate to serve as a biocatalyst in the conversion of glucosamine to glycosaminoglycans.

Thus, the present invention provides unique combinations of the necessary precursors to articular cartilage regeneration. When taken as directed, and in accordance with the treatments set forth herein, the compositions of the present invention aid in the reparation of damaged articular cartilage and stimulates the growth of new articular cartilage; providing symptomatic relief from pain and inflammation associated with articular cartilage degeneration.

In one embodiment the compositions of the present invention comprise nutritional supplements useful as biocatalysts which are chondroitin sulfate, glucosamine sulfate potassium, glucosamine sulfate sodium, glucosamine hydrochloride, N-Acetyl D-Glucosamine, methylsulfonylmethane, ascorbate Sodium, and chelated manganese proteinate.
1) Chondroitin Sulfate is a long hydrophilic chain of repeating sugars. This glycosaminoglycan binds to proteoglycan molecules aiding in water and nutrient transportation within the articular cartilage. Chondroitin in its sulfate form includes galactosamine, a primary substrate of hyaluronan and a disaccharide pathway for proteoglycan synthesis secondary to the hexosamine pathways utilized for glycosaminoglycan production. Chondroitin sulfate chains comprise the space formation of the cartilage matrix and integral parts of the proteoglycan molecule. Chondroitin stimulates the production of proteoglycans, glycosaminoglycans, and collagen, which are the building blocks of healthy cartilage. Chondroitin also inhibits the secretion of degenerative enzymes by the chondrocytes within articular cartilage. Chondroitin Sulfates are non-toxic and work synergistically with glucosamine to hydrate and repair articular cartilage. Presently preferred compositions and treatments provided by the invention can comprise from about 2 mg to about 6 mg of chondroitin sulfate per pound of body weight depending upon the species being treated and can further be case dependent.
2) Glucosamine is an amino sugar comprised of glucose and an amino acid glutamine. It is an important part of mucopolysaccharides, which provide structure to bone, cartilage, skin, nails, hair, and other body tissues. The presence of glucosamine with the cartilage matrix stimulates the chondrocytes to produce collagen and proteoglycans, the major components of articular cartilage.
   A) Glucosamine Sulfate Sodium is an aminosugar bound to a composition of sulfate sodium and is a primary substrate for collagen and proteoglycan production. Specifically glucosamine sulfate form is a biocatalyst in the conversion of glucosamine to glycosaminoglycans. Presently preferred compositions and treatments provided by the invention can comprise from 2 mg to 6 mg of chondroitin sulfate per pound of body weight being species dependent.
   B) Glucosamine Sulfate Potassium is an amino sugar composition bound to the mineral potassium. Glucosamine potassium facilitates cellular membrane function while normalizing the sodium/potassium ratio. In turn glucosamine potassium aids in the exchange of water, nutrients, and waste products allowing the desired introduction of glucosamine for proteoglycan synthesis. Presently preferred compositions and treatments provided by the invention can comprise from 2 mg to 6mg glucosamine potassium per pound of body weight being species dependent.
   C) Glucosamine hydrochloride is an amino sugar derivative through chemical hydrolysis of hydrochloric acid on disaccharides making it readily bioavailable for the production of hyaluronic acid. In this form glucosamine binds to glucuronic acid directly facilitator and stimulating the chondrocytes production of hyaluronic acid. Presently preferred compositions and treatments provided by the invention can comprise from 2 mg to 6 mg glucosamine hydrochloride per pound of body weight being species dependent.
   D) N-Acetyl D-Glucosamine is a derivative of glucose obtained by chemical hydrolysis of chitin. This polysaccharide is readily soluble in water and extremely bioavailable. N-Acetyl D-glucosamine binds to glucuronic acid as well as galactose making it a precursor to hyaluronic acid, keratan-sulfate and chondroitin sulfate. This unique derivative aids proteoglycan, collagen and glycosaminoglycan production. N-acetyl D-glucosamine has also been shown to aid in the healing of soft tissue injury. Presently preferred compositions and treatments provided by the invention can comprise from 0.6 mg to 1 mg N-Acetyl D-Glucosamine per pound of body weight being species dependent.
3) Methylsulfonylmethane: (MSM) is a naturally occurring bioavailable form of sulfur composed of methionine, cysteine, taurine, and glutathione. MSM's unique amino acid profile assists in the delivery of nutritional components to tissue and is necessary for the synthesis of collagen. Its components regulate glucose uptake while assisting in the formation of disulfide bonds which hold molecular strands of connective tissues together. Glutathione provides antioxidant protection within the cartilage matrix. An essential role of MSM is determining the contour of diverse biomolecules and is essential to the activity of many enzymes that protect and sustain protoplasm. Presently preferred compositions and treatments provided by the invention can comprise from 4 mg to 10 mg MSM per pound of body weight being species dependent.
4) Sodium Ascorbate is an electrolyte bound absorbatc providing antioxidant properties. Ascorbate is an essential co-factor as part of an enzyme system. It is responsible for many biochemical functions including 1) Maintenance and repair of all connective tissues especially the collagen component. 2) Biosynthesis of bone, teeth, and cartilage. 3) Enhancing manganese utilization within the body. Presently preferred compositions and treatments provided by the invention can comprise from 2 mg to 3 mg sodium ascorbate per pound of body weight being species dependent
5) Manganese Proteinate: A peptide bound form of the mineral manganese, which plays an important role in the synthesis of glycosaminoglycans, and glucoproteins. Manganese works synergistically with glucosamine and chondroitin sulfate to produce hyaluronic acid, by-passing the rate limiting steps of proteoglycan and collagen production. Independently it is essential for the maintenance and repair of connective tissue. Presently preferred compositions and treatments provided by the invention can comprise from 2 mg to 3 mg manganese proteinate per pound of body weight being species dependent.

The embodiments of the presents invention have proven safe and non toxic in the prescribed amounts. Each embodiment provides a specific benefit in relation to the repair and regeneration of articular cartilage. Thus, it can be realized that the compositions of this invention comprised of chondroitin sulfate, glucosamine sulfate sodium, glucosamine hydrochloride, glucosamine sulfate potassium, N-acetyl D-glucosamine, methylsulfonylmethane, ascorbate and manganese proteinate provide a unique combination of ample metabolic precursors which advantageously stimulate the production of glycosaminoglycans including hyaluronic acid, proteoglycans and collagen, thereby assisting the body's natural repair mechanisms and specifically directing the chondrocytes in the production of new articular cartilage. It may also be realized that the presence of glucosamine potassium and MSM aid in cellular membrane function encouraging nutritional uptake and waste removal within the articular cartilage. It may also be realized that such a composition administered orally or paternally is digestible and assimilated in mammals. It may also be realized that the composition of this invention is capable of symptomatic relief of pain and inflammation associated with articular cartilage degeneration.

### Examples of the Invention

The following case studies were conducted with mammals. Symptomatic relief in animal behavior and recovery from afflictions demonstrates the efficacy of the composition.

**Case Study No.1:** An eleven year old male pure bred German Shepherd weighing 95 pounds presented symptoms of difficulty rising in rear and "sinking" of the hind quarters. Further this shepherd became reluctant and soon refused to participate or initiate the game of retrieval with a tennis ball. Physical examination revealed pain when hips were palpated. Preliminary diagnosis was hip dysplasia. Upon recommendation by veterinarian, a regiment of COSEQUIN® (U.S. Patent No. 5,364,845 to Henderson) was administered for a period of 60 days improvement in symptoms were insignificant. Difficulty rising and refusal to retrieve were still evident. Cosequin treatments were discontinued and a regiment of the present invention was introduced. Including 600mg. MSM, 380mg. chondroitin sulfate, 380mg. glucosamine potassium, 380mg. glucosamine hydrochloride, 380mg. glucosamine sulfate, 100mg. N-acetyl D-glucosamine, 100mg. ascorbate and 30mg. manganese Proteinate divided into two doses administered paternally with dog food morning and evening. Within 14 days of initial administration significant improvement in mobility and gait were apparent, interest in retrieval became routine. On day 30 palpation by the veterinarian revealed no pain or discomfort. Relief from symptoms was directly attributed to the introduction of the present invention into the dog's diet.

**Case Study No. 2:** A ten year old cat weighing ten pounds presented symptoms of a slight limp in hind right leg and refusal to jump onto couch or bed as previously displayed. Veterinarian palpation revealed pain in hind hip and stiff joint. Preliminary diagnosis being degenerative joint disease. A regiment of the present invention was introduced including 70 mg. MSM, 40mg. chondroitin sulfate, 40mg. glucosamine potassium, 40mg. glucosamine hydrochloride, 40mg. glucosamine sodium, 10mg. N-Acetyl D-Glucosamine, 10mg. sodium ascorbate, 3mg. manganese proteinate. Divided into two doses administered paternally with cat food morning and evening. Within 7 days of initial administration symptoms of a limp had vanished, gait had returned to normal. Within 21 days of initial administration the behavior of jumping on couches and beds had returned. Cat remained on present invention for 60 days when administration was halted. After 120 days of initial administration, no prior symptoms of DJD were detectable. Alleviation of symptoms and longevity of relief attributed to use of present invention.

**Case Study No. 3:** An 18 year old thoroughbred retired after racing career. Currently a broodmare weighing approximately 1200 lbs. diagnosed by veterinarian with ring bone in hind pastern and degenerative joint disease in knees displayed tactile symptoms of heat and inflammation, while exhibiting difficulty walking and trotting. Was placed on a regiment of the present invention after aborting pregnancy due to increased load bearing stress. While pregnant, this mare's symptoms worsened in direct relation to the growth of foal. By the 5^{th} month of pregnancy, injections of ADAQUAN® (an intravenous GAG) were administered; mild symptomatic relief was evident but short lived. By the 7^{th} month she was unable to walk and refused to eat, resulting in stress related abortion of the foal. Subsequently the mare was examined and no uterine abnormalities were detected. The current invention was introduced paternally including 4g. MSM, 2.4g.glucosamine potassium, 2.4g. chondroitin sulfate 2.4g.glucosamine hydrochloride, 2.4g.glucosamine sulfate, 0.6g. N-Acetyl D-Glucosamine, 0.6g. sodium ascorbate, 0.2g. manganese proteinate. Three months prior to breeding and remained on this regiment through foaling. After twenty days of initial administration outward signs of discomfort walking and trotting had subsided. After forty days of initial administration, tactile symptoms of heat and inflammation in the joint areas had dissipated as well. The mare remained on the present invention through foaling without reoccurrence of symptoms. The mare currently remains on the same regiment. The introduction of the present invention into the diet of this mare was directly responsible for increased mobility, pain management, and joint reparation.

**Case Study No. 4:** A 5 year old gelding thoroughbred race horse weighing approximately 1000 pounds retired early due to trauma of the knees. Veterinarian diagnosis confirmed DJD due to trauma resulting in loss of articular cartilage. This race horse was placed in the Re-Run thoroughbred relocation program yet was un-adoptable due to its inability to support a rider. Tactile symptoms of heat and inflammation were present, walking and trotting were labored and he refused to run with other horses in field. The present invention was introduced including 4g. MSM 2.4g. chondroitin sulfate/, 2.4g. glucosamine potassium, 2.4g. glucosamine hydrochloride, 2.4g. glucosamine sulfate .6 N-Acetyl D-Glucosamine, 0.6g. sodium ascorbate, 0.2g. manganese proteinate, paternally as a top dressing. Within 14 days of administration heat and inflammation were absent in palpation, walking and trotting appeared normal and brief sprints with the herd were observed. After 30 days re-examination by the veterinarian revealed no pain during palpation. Symptoms of heat and inflammation had disappeared. Behavior in field was observed as that of normal including integrating and running with the herd. No visual symptoms of lameness were apparent. Re evaluation of his status was made, and the gelding was deemed sound for rider and adoptable. The introduction of present invention was directly responsible for reparation of the articular cartilage in the knees caused by trauma and reversal of related symptoms.

The foregoing examples and case studies have been presented to demonstrate the efficacy of the composition of the current invention. Alleviation and reversal of symptoms related to the degeneration of articular cartilage in these studies is testimony to the direct effect the present invention has in recovery from per-existing afflictions. The resultant benefits can only be attributed to the current inventions ability to repair and stimulate the regeneration of articular cartilage. In each case the introduction of the present invention was paternal and the animals were unaware that treatment was being administered, thus ruling out the "placebo effect." In each case, introduction of the present invention was identified as the only management variable in the animal's routine. Similar results have been documented with humans, yet clinical trials are unavailable.

## Claims

1. A composition comprised of synergistic nutritional supplements useful for repair of articular cartilage in a mammal, comprising therapeutic amounts of: methylsulfonylmethane; chondroitin sulphate; glucosamine sulphate potassium; glucosamine sulphate sodium; glucosamine hydrochloride; N-Acetyl D-Glucosamine; sodium ascorbate, and chelated manganese proteinate.

2. The composition of Claim 1 wherein the composition stimulates chondrocytes to produce proteoglycans, glycosaminoglycans, hyaluronan, and collagen which repair and rebuild articular cartilage.

3. The composition of Claim 1 wherein the composition inhibits degenerative enzymes within the articular cartilage.

4. The composition of Claim 1 wherein the composition aids in nutrient transportation within cells of the articular cartilage matrix.

5. The composition of Claim 1 wherein the composition provides an optimal delivery system for the present composition as a biocatalyst, elevating and sustaining blood glucosamine and chondroitin levels, making these components more bioavailable then previous compositions.

6. The composition of Claim 1 wherein the composition provides the necessary components to stimulate synovial cells synthesis of hyaluronic acid.

7. A composition useful for repair of articular cartilage in a mammal, comprising a therapeutic amount of glucosamine sulphate potassium and a therapeutic amount of methylsulfonylmethane.

8. The composition of Claim 7, further comprising at least one synergistic nutritional supplement selected from the group consisting of chondroitin sulfate; glucosamine sulphate sodium; glucosamine hydrochloride; N-Acetyl D-Glucosamine; sodium ascorbate, chelated manganese proteinate and combinations thereof.

9. The composition of Claim 7, wherein the therapeutic amount is from between 4 mg to 10 mg of methylsulfonylmethane per pound of body weight per unit dose.

## Patentansprüche

1. Zusammensetzung, umfassend synergistische Nahrungsergänzungen, die zur Wiederherstellung von Gelenkknorpel in einem Säuger geeignet ist, wobei die Zusammensetzung therapeutische Mengen von Methylsulfonylmethan, Chondroitinsulfat, Glukosaminsulfat-Kalium, Glukosaminsulfat-Natrium, Glukosaminhydrochlorid, N-Acetyl-D-glukosamin, Natriumascorbat und chelatisiertes Manganproteinat umfasst.

2. Zusammensetzung nach Anspruch 1, wobei die Zusammensetzung Knorpelzellen stimuliert, Proteoglykane, Glykosaminoglykane, Hyaluronan und Kollagen zu erzeugen, die Gelenkknorpel wiederherstellen und neu aufbauen.

3. Zusammensetzung nach Anspruch 1, wobei die Zusammensetzung degenerative Enzyme innerhalb des Gelenkknorpels inhibiert.

4. Zusammensetzung nach Anspruch 1, wobei die Zusammensetzung den Nährstofftransport innerhalb von Zellen der Gelenkknorpelmatrix unterstützt.

5. Zusammensetzung nach Anspruch 1, wobei die Zusammensetzung ein optimales Abgabesystem für die vorliegende Zusammensetzung als Biokatalysator bereitstellt, der die Glukosamin- und Chondroitin-Blutkonzentrationen erhöht und aufrechterhält, wodurch diese Komponenten eine bessere Bioverfügbarkeit aufweisen als bei bisherigen Zusammensetzungen.

6. Zusammensetzung nach Anspruch 1, wobei die Zusammensetzung die erforderlichen Komponenten zur Stimulation der Synthese von Hyaluronsäure durch Synovialzellen bereitstellt.

7. Zusammensetzung, die zur Wiederherstellung von Gelenkknorpel in einem Säuger geeignet ist, umfassend eine therapeutische Menge an Glukosaminsulfat-Kalium und eine therapeutische Menge an Methylsulfonylmethan.

8. Zusammensetzung nach Anspruch 7, die ferner mindestens eine synergistische Nahrungsergänzung umfasst, die aus der Gruppe bestehend aus Chondroitinsulfat, Glukosaminsulfat-Natrium, Glukosaminhydrochlorid, N-Acetyl-D-glukosamin, Natriumascorbat, chelatisiertes Manganproteinat und Kombinationen davon ausgewählt ist.

9. Zusammensetzung nach Anspruch 7, bei der die therapeutische Menge zwischen 4 mg und 10 mg Methylsulfonylmethan pro Pfund Körpergewicht pro Einheitsdosis beträgt.

## Revendications

1. - Composition comprenant des suppléments nutritionnels synergiques utiles pour la réparation de cartilage articulaire dans un mammifère, comprenant des quantités thérapeutiques de : méthylsulfonylméthane ; chondroïtine sulfate ; glucosamine sulfate de potassium ; glucosamine sulfate de sodium ; chlorhydrate de glucosamine ; N-Acétyl-D-Glucosamine ; ascorbate de sodium et protéinate de manganèse chélaté.

2. - Composition selon la revendication 1, dans laquelle la composition stimule les chondrocytes à produire des protéoglycanes, des glycosaminoglycanes, de l'hyaluronane et du collagène qui réparent et reconstruisent le cartilage articulaire.

3. - Composition selon la revendication 1, dans laquelle la composition inhibe les enzymes dégénératives à l'intérieur du cartilage articulaire.

4. - Composition selon la revendication 1, dans laquelle la composition aide au transport des nutriments à l'intérieur des cellules de la matrice du cartilage articulaire.

5. - Composition selon la revendication 1, dans laquelle la composition fournit un système d'administration optimal pour la présente composition en tant que biocatalyseur, élevant et maintenant les taux de glucosamine et de chondroïtine dans le sang, rendant ces composants plus biodisponibles que les compositions précédentes.

6. - Composition selon la revendication 1, dans laquelle la composition fournit les composants nécessaires pour stimuler la synthèse des cellules synoviales de l'acide hyaluronique.

7. - Composition utile pour réparer le cartilage articulaire dans un mammifère, comprenant une quantité thérapeutique de glucosamine sulfate de potassium et une quantité thérapeutique de méthylsulfonylméthane.

8. - Composition selon la revendication 7, comprenant en outre au moins un supplément nutritionnel synergique choisi dans le groupe constitué par le chondroïtine sulfate, le glucosamine sulfate de sodium, le chlorhydrate de glucosamine, le N-Acétyl-D-Glucosamine, l'ascorbate de sodium, le protéinate de manganèse chélaté et les combinaisons de ceux-ci.

9. - Composition selon la revendication 7, dans laquelle la quantité thérapeutique est d'entre 4 mg et 10 mg de méthylsulfonylméthane par livre de poids corporel par dose unitaire.
